# EUROPEAN PATENT SPECIFICATION

(11) **EP 2 887 977 B1**
(45) Date of publication and mention of the grant of the patent: **30.12.2020**
(21) Application number: 13762636.2
(22) Date of filing: 27.08.2013
(51) Int. Cl.: A61M 1/02

(54) **APPARATUS AND METHOD FOR SEPARATION AND CONCENTRATING FLUIDS CONTAINING MULTIPLE COMPONENTS**
VORRICHTUNG UND VERFAHREN ZUM TRENNEN UND KONZENTRIEREN VON FLÜSSIGKEITEN MIT MEHREREN KOMPONENTEN
APPAREIL ET PROCÉDÉ POUR SÉPARER ET CONCENTRER DES FLUIDES CONTENANT DE MULTIPLES COMPOSANTS

(30) Priority: 27.08.2012 US 201213595461
(43) Date of publication of application: 01.07.2015
(73) Proprietor: Biomet Biologics, LLC, Warsaw, Indiana 46582 (US)
(72) Inventor: LANDRIGAN, Matthew D., Fort Wayne, Indiana 46814 (US); SWIFT, Matthew, Fort Wayne, Indiana 46818 (US)
(74) Representative: Handley, Matthew Edward
(86) International application number: PCT/US2013/056793
(87) International publication number: WO 2014/035964

(56) References cited:
- WO-A1-2006/041406
- WO-A1-2008/127639
- US-A1- 2010 140 182
- US-A1- 2010 256 595

## Description

### FIELD

The subject disclosure is related to separating a whole material including multiple components into selected fractions of components, and particularly to a method and apparatus for allowing a float member to operate generally unhindered in a container.

### BACKGROUND

This section provides background information related to the present disclosure which is not necessarily prior art Examples of prior art can be found in documents US 2010/140182 and WO 2008/127639.

Clinicians have identified a wide range of therapeutic and laboratory applications for autologous isolated fractions, such as platelet concentrate, platelet-poor-plasma, and stromal cells, of suspensions such as blood, bone marrow aspirate, and adipose tissue. Clinicians generally prefer to draw and fractionate the autologous suspension at the point-of-care. Point-of-care fractionation can reduce the need for multiple appointments to draw and fractionate the autologous suspension which can be costly and inconvenient. Additionally, point-of-care preparation reduces potential degradation of the autologous suspension that can begin once the autologous suspension is removed from a patient. Point-of-care fractionation systems should be easy to operate to reduce the need to provide clinicians with extensive instruction, quick so the therapeutic fraction can be isolated and administered during a single patient visit, efficient to effectively isolate the fraction to a desired concentration, and reproducible to operate over wide variations in suspension characteristics. An example of a buoy based suspension fractionation system is shown in Biomet Biologics, Inc. international brochure entitled *"*Gravitational Platelet Separation System Accelerating the Body's Natural Healing Process," 2006.

### SUMMARY

This section provides a general summary of the disclosure, and is not a comprehensive disclosure of its full scope or all of its features.

Whole materials can be extracted from various sources, such as a human body or banked materials. For example, whole blood can be withdrawn from a patient or be withdrawn from a blood bank. The whole material, however, can be separated into various components thereof in fractions based upon density and gravity separation. For example, the whole material can be positioned in a separation container and centrifuged to fractionate or separate into fractions the various components in the whole blood. Fractions can include red blood cells, plasma, and a buffy coat including platelets, white blood cells, stromal cells, and various growth factors. The whole material can also include bone marrow, a mixture of blood and bone marrow, adipose, and other materials.

A buoy or float can be positioned within the separation container to assist in a physical separation of the whole material positioned in the separation container. The buoy can be positioned within the container to move between a first end and a second end. The buoy can also provide a physical separation of the first end and the second end. The buoy can include or be formed to have a selected specific gravity or density such that it will move to a selected location within a fractionated whole material so that a portion of the buoy, such as a middle collection area, of the buoy is positioned at a selected location within the fractionated material.

The separation container can include an opening or port into the container for introduction of a whole material which can also be referred to as a multiple component material. The container can include at least one withdrawal port at or near a second end opposite the first end and on a second and opposite side of the buoy for withdrawal of at least a portion of the whole material. A withdrawal tube can also be positioned between at least of one port at the second end of the separation container for connection to the buoy system to allow withdrawal from a middle region or middle collection portion of the buoy. The first portal on the first end of the container can also include a valve, such as a check valve, to limit a direction of flow of material into the separation container.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Fig. 1 is an environmental view of a separation container;
Fig. 2 is an exploded view of the separation container of Fig. 1;
Fig. 3 is a cross-sectional view of the separation container of Fig. 1 along line 3-3;
Fig. 4A is a partial cross-sectional view of the separation container;
Fig. 4B is a partial cross-sectional view of a separation container according to various embodiments;
Fig. 5 is an environmental view of a process of withdrawing at least a portion of a whole material from a patient;
Fig. 6 is a partial cross-sectional view of filling the separation container;
Fig. 7 is a cross-sectional view of the separation container filled with a selected whole material; and
Fig. 8 is a cross-sectional view of a whole material that has been separated where at least a component thereof is being withdrawn from the separation container.

Corresponding reference numerals indicate corresponding parts throughout the several views of the drawings.

### DETAILED DESCRIPTION

Example embodiments will now be described more fully with reference to the accompanying drawings.

A separation container 20 can generally include a sidewall 22 that extends from a first end 24 to a second end 26. The first end 24 can include a first end wall 25 and/or be covered or connected to a stand 28. The stand 28 can assist in maintaining the container 20 in an upright position or vertical position when the container 20 is not positioned in a centrifuge, as discussed further herein. The sidewall 22 extending to the second end 26 can include a second end wall and/or be capped or covered by a cap 30. The cap 30 can be separate and connected to the wall 22 or formed as one piece therewith. Extending through the cap 30 at the second end 26 can be a first port 32 that is covered by a port cap 34 and a second port 36 that is covered by a second port cap 38. In addition, a vent 40 can be formed through the cap 30 that can include a selected filter material such that the interior of the container 20 remains substantially sterile. The vent 40 can be similar to the vent and included filter material in the GPS® II Blood Separation Device, sold by Biomet, Inc., having a place of business in Indiana.

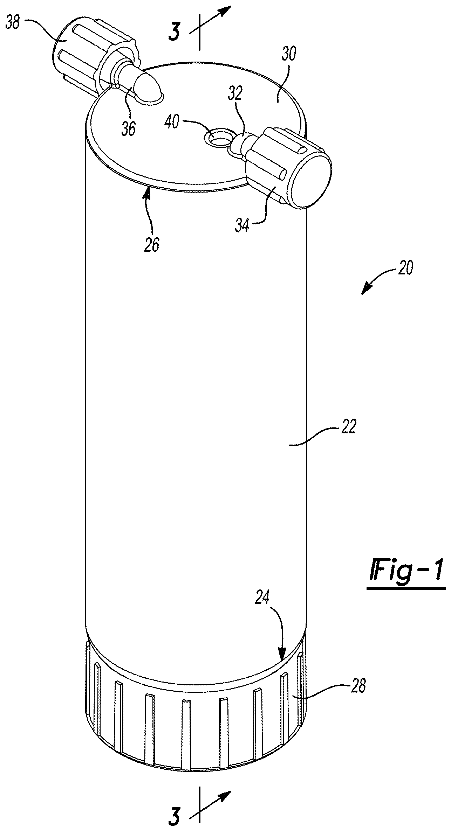

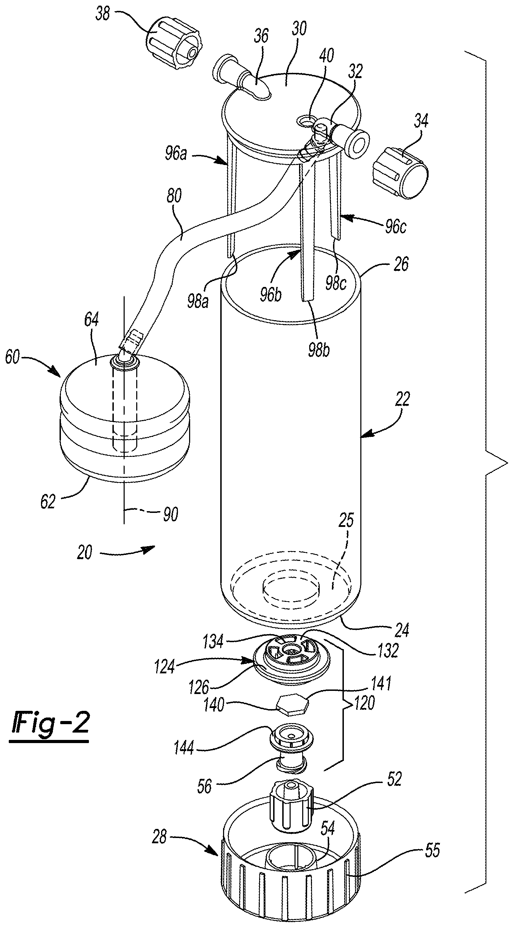

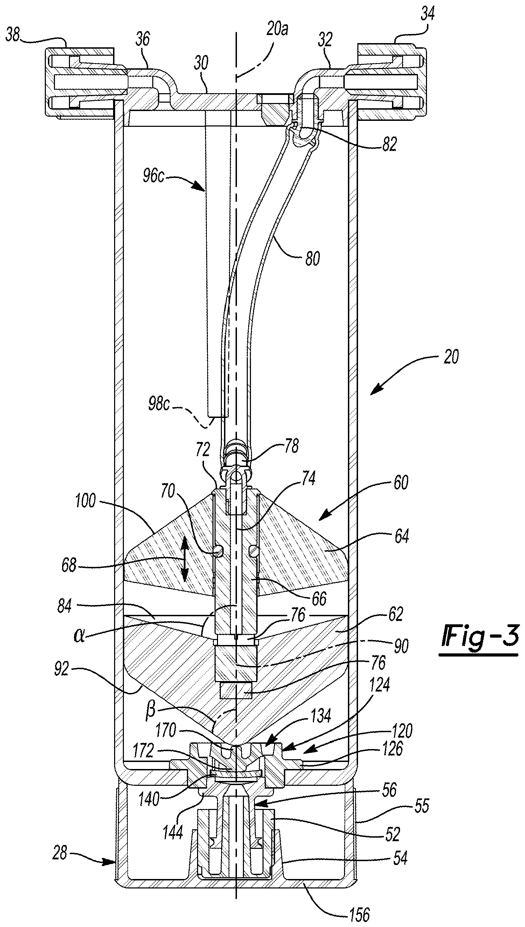

With continuing reference to Fig. 1 and additional reference to Figs. 2 and 3, the separation container 20 can be provided by a manufacturer substantially assembled or in multiple pieces, as illustrated in exploded view in Fig. 2. Nevertheless, the stand 28 can be removed or removable to be interconnected with a port, including a luer type port 56 or a cap 52 that interconnects with the luer type connector 56. In particular, the cap 52 can include an exterior wall that can be engaged or coupled to an extending wall or connection portion 54 of the stand 28. An outer extending wall 55 of the stand 28 can be formed to include an outer diameter or dimension that is substantially equivalent to the dimension of the wall 22 of the container 20. The internal area or portion of the stand 28 can therefore be substantially hollow save for the extending or connection portion 54 that engages the cap 52. The interconnection of the stand 28 and the cap 52 can be a friction or interference fit, glued, or can include other connection portions such as a thread or taper. Also, the stand 28 can be formed integrally or one piece, such as to include, the cap 52. In other words, the upstanding wall 54 can include a luer connection to engage the luer type connector 56.

The stand 28 is generally provided to hold the container 20 in a vertical position or upright orientation when placed on a surface. Further, the stand 28 is generally provided to allow the container 20, with the stand 28 in place on the container 20, to be positioned within a bucket of a centrifuge system, and to support the container 20 against centrifugal forces during centrifugation. For example, the stand 28 can contact an edge near the first end 24 to support the container 20 within a centrifuge bucket.

Positionable within the container 20 is a buoy or float system 60. The buoy 60 can include a first buoy member 62 and a second buoy member 64. Interconnecting the first and second buoy members 62, 64 can be a third buoy member or post 66. The third buoy member 66 can allow or have the second buoy member 64 slide along or move along the buoy post 66 generally in the direction of arrow 68. The second buoy member 64 can be sealed relative to the buoy post 66 with an appropriate sealing portion such as a tight fit or an O-Ring 70. A lip or shoulder 72 can limit travel of the second buoy member 64 relative to the first buoy member 62 and the buoy post 66.

Additionally, the buoy post can define a collection passage 74 that includes collection face passages 76. The collection passage 74 can pass through a port or hose connection member 78 to which a hose or tube 80 is connected. The hose 80 can be connected to a second hose connection 82 that can extend from the first port 32. As discussed herein, a member can be interconnected with a first port 32 to allow withdrawal of a material from a collection face or surface 84 of the first buoy member 62 by withdrawing material through the collection passage 74, tube 80, and first port 32. The member can include a tube connection, a syringe, or other appropriate connectable member.

The buoy 60 can be a buoy that is substantially similar to the buoy disclosed in U.S. Pat. No. 8,067,534 or sold in the GPS® II Blood Separation Device, sold by Biomet, Inc. In addition, the buoy system 60 can be similar to the buoy system disclosed in U.S. Pat. No. 7,806,276 presently sold in the GPS® III Blood Separation Device, sold by Biomet, Inc. Accordingly, the buoy system can have a total density of about 1.01 grams per cubic centimeter (g/cm³) to about 1.10 g/cm³. Further, the first buoy member 62 can have a first density different from a second density of the second buoy member 64. Thus, the first buoy member 62 can have the first density of about 1.02 g/cm³ to about 1.10 g/cm³ and the second buoy member 64 can have the second density of about 0.90 g/cm³ to about 1.01 g/cm³. This can assist in allowing the second buoy member 64 to slide along the buoy post 66 during separation of material positioned in the container 20.

The buoy system 60 can generally define a central axis or line 90. It can be selected to assemble the buoy 60 into the container 20 such that the central axis 90 of the buoy 60 is aligned with the longitudinal axis 20a of the container. The collection face 84 can define an angle α relative to the central axis 90 and the buoy bottom or second surface 92 can define an internal angle β relative to the central axis 90. It is understood that a complimentary angle can also be defined from the bottom surface or wall 92 to a line that is substantially normal to the central axis 90. The angle α can be about 55 to about 85 degrees. The angle β can be about 30 to 85 degrees, including about 55 degrees. The angle β can be selected to assist passage of at least a portion of material placed in the container 20 to pass the first buoy member 62. That is, the less the angle β (i.e. the steeper the angle β) the easier for material to pass the first buoy member 62.

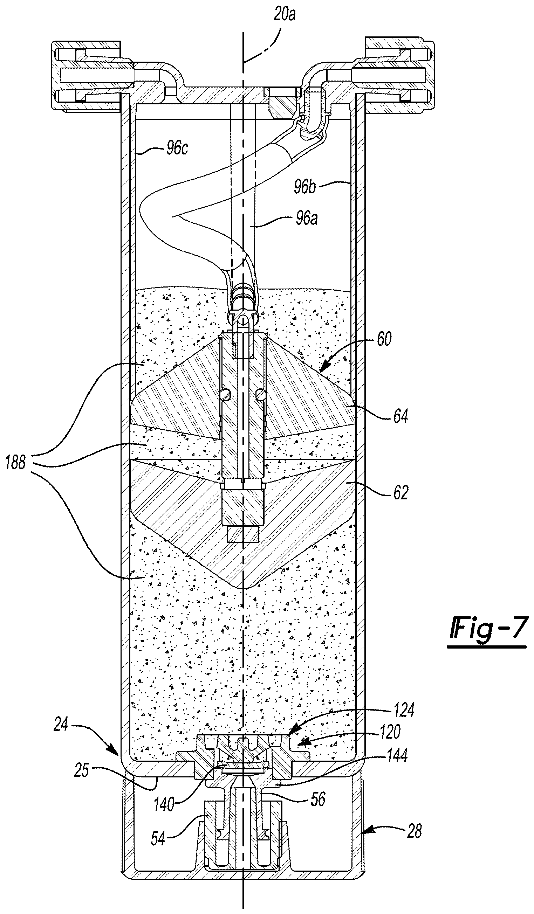

The cap 30 can be positioned on the top of the wall 22 to substantially close and/or seal the container 20. Extending from the cap 30 can be stop legs or members 96a-96c. The legs 96a-96c extend from the cap to respective distal ends 98a-c. The distal end of the legs 98a-98c can engage a top surface 100 of the second buoy member 64, as illustrated in Fig. 7, to limit a movement of the buoy member 60 towards the cap 30 during loading and at least partial filling of the container 20 with a fluid, including a biological material 188, a separation, or centrifugation operation of a whole material within the container 20. The limited movement can be predetermined and can be about 30% to about 70%, including about 66% of the total length of the container 20. Thus, the movement of the buoy 60 can be limited within the container 20 when the cap 30 is in place by the legs 96a-96c extending therefrom.

As discussed further herein, the legs 96a-96c can be set at a fixed location relative to one of the ends of the container 20 such that the maximum movement of the buoy system 60 can be maintained within the container 20. It will also be understood, however, that the legs 96a-c can be provided in any appropriate number, such as one leg may be able to provide an appropriate abutment surface area to stop the buoy at a selected location. Additionally, the legs 96a-c can be provided as projections or protuberances from the container 20 that extend into the container 20 that are substantially transverse to a long axis of the container 20.

The container 20, as discussed above, extends from the first end 24 to the second end 26. At the second end 26, the cap 30 can engage the second end or can be formed integrally therewith. At the first end 24, the bottom wall or first end wall 25 can extend from the sidewall 22. The first end wall 25 can further include a valve assembly 120 or at least a portion thereof. The valve assembly 120 allows the container to be filled through the first end 24 while the ports 32, 36 allow the container to be emptied or have material withdrawn from the second end 26. It is understood that the first end 24 may be a bottom end and the second end 26 may be a top end.

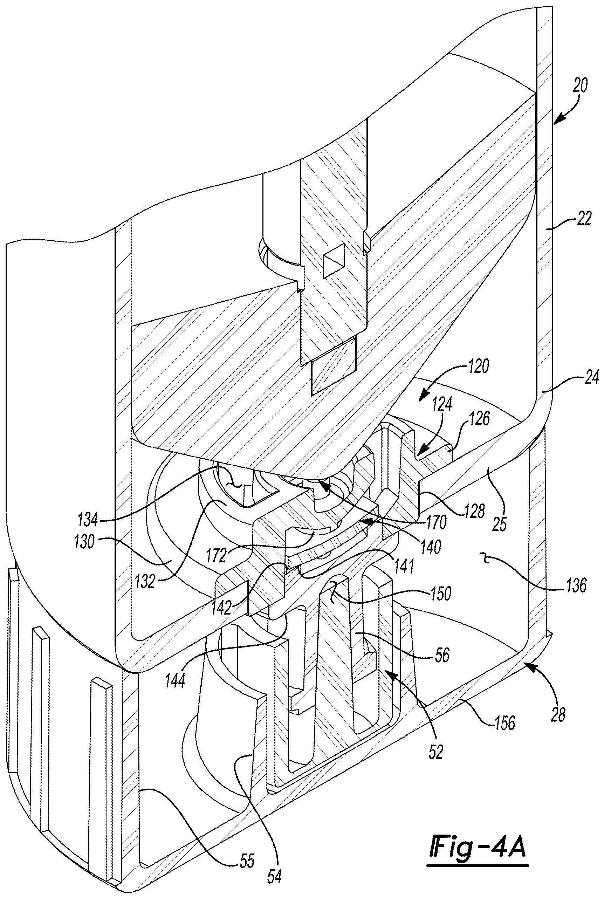

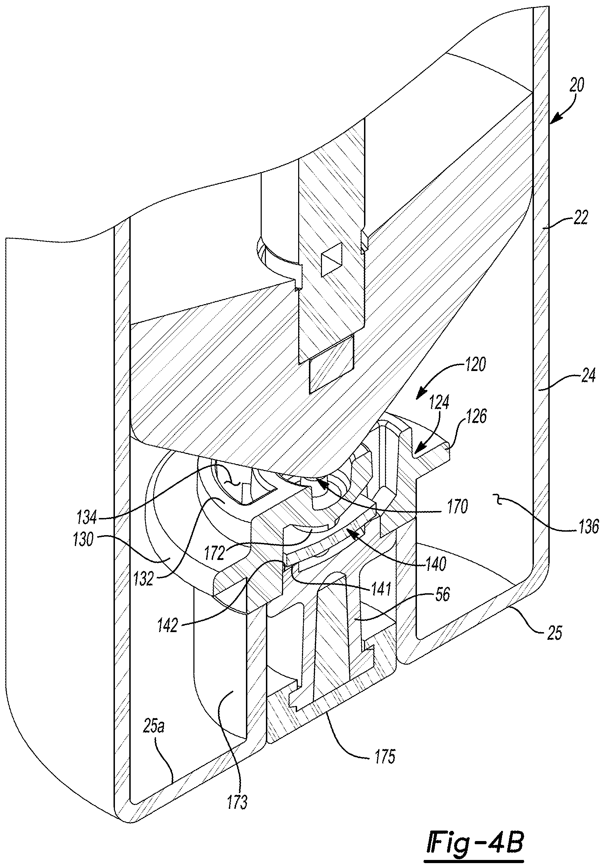

The valve assembly 120 can include an outer wall or body portion 124 that includes a flange 126 that extends external to an outer wall portion 128 of the valve body 124 to engage the first end wall 25. It will be understood, however, that the valve assembly 120, including the valve body portion 124, can be formed as a single piece with the first end wall 25. However, as illustrated in Fig. 4, the valve assembly 120 can be formed separately and connected to the first end wall 25. Appropriate connections can include adhesives, welding, snap or interference fitting, or any other appropriate connection. The valve body 124 generally will be connected with the first end wall 25 in a substantially liquid tight manner. Accordingly, a fluid positioned within the container 20 will not leak around the valve body 124 to an area external to the container 20. Nevertheless, the valve assembly 120 can be operated to allow a material to be at least placed within the container 20 from a position external to the container 20, as discussed further herein.

With continuing reference to Figs. 1-3 and additional reference to Fig. 4, the valve body 124 can include the outer wall portion 128 that extends through the first end wall 25 and a second outer wall portion 130 that extends opposite the first outer wall portion 128 on the opposite side of the flange 126. The second outer wall portion 130 can extend to a top portion and a top wall 132 can be defined substantially transverse to the outer wall portion 130. The top wall portion 132 can be a transverse wall and can define passages 134 that extend axially through the valve body 124 from an area exterior to the container 20, such as an area below or outside of the first end wall 25 generally defined as stand enclosed area 136. The area 136, as illustrated in Fig. 4, can be enclosed by a stand or end cap 28 as discussed further herein. Nevertheless, the valve passage 134 can be defined as a passage through the valve body 124 that is closed by a valve or flexible valve member 140. The flexible valve member 140 can seat on an upper edge 142 of a second valve body portion 144 that is interconnected with the valve body 124 and a lower edge 172 of the first valve body 124. The interconnection of the second valve body 144 with the valve body 124 can be any appropriate connection, such as an adhesive, welding, or the like. The second valve body 144 can also be formed as one piece with the luer member 56 or connected thereto.

The check valve member 140 can be formed of any appropriate flexible but sealing material. For example, silicon or other natural or synthetic rubbers can be used as the sealing member 140. Generally the valve or sealing member 140 can be formed of a biocompatible material that will not contaminate a biological material, such as white blood cells, stromal cells, red blood cells, platelets, plasma, or other biological materials. Additionally, the valve member 140 can be flexible without being toxic to a patient.

The flexible valve body 140 can define a valve member or closing portion between the passage 134 defined by the first valve body 124 and a second valve passage 150 defined by the second valve body 144. The second valve passage 150 can be formed through the second valve body 144 where a portion of the second valve body 144 can define the luer fitting 56. It will be understood that other appropriate connections can be provided other than the luer fitting 56, but luer fittings are a generally known connection. The sealing cap or luer cap 52 can be provided to interconnect with the luer fitting 56 to seal or otherwise cover the second valve body 144. The stand 28 can interconnect with the cap 52 by an interference, snap-fit connection, or with glue. The stand 28 can include a bottom wall 156 and the sidewall 55 to contact the first end wall 25 of the container 20. The stand 28, therefore, can assist in holding the container 20 in a substantially vertical position although the second valve body 144 and the luer cap 52 extend and provide a small surface area from the bottom of the container 20. It will be understood that the stand 28 can also be formed integrally or as one piece with the cap 52.

The valve body 124 can also include a boss or contact point 170 to engage the buoy 60, and particularly at a point or bottom surface 92 of the buoy member 62. It will be understood, however, that the surface 132 can be substantially flat save for the passages or passage 134 defined through the valve body 124. The valve assembly 120, therefore, can provide or form a contact surface with the buoy 60 to resist movement of the buoy 60 and to maintain the buoy within the container 20.

The valve member 140 can be fixed within the valve assembly 120 such that it is able to flex relative to the first valve body 124. In particular, the valve member 140 can be a check valve that allows for a material to enter the container 20 while resisting or substantially limiting material removal or movement through the check valve 120 out of the container 20. Therefore, the flexible member can generally include an edge 141, such as an annular or other geometric shaped edge, that engages or contacts an edge 142 of the second body 144. In a relaxed or closed position the check valve member 140 is resting on the edge 142 and substantially closes the valve assembly 120. The valve member 140 can flex towards the surface 132 either completely or at an edge. If only an edge flexes a central portion of the valve member 140 can be held in place by a boss portion 172. Accordingly, the valve member 140 can flex around an edge, such as an annular edge, near the edge 142 of the second valve body 144 to allow material to pass into the container 20, as discussed further herein.

Although, as discussed above, the valve assembly 120 can include at least one valve body portion 144 and/or luer connection 56 that is external to the end wall 25, it will be understood that the end wall 25 may be the external most portion of the container 20. That is, the valve assembly 120 can be formed to seat internally of the end wall 25 such that the external surface of the end wall 25 is flush and/or substantially planar, as illustrated in Fig. 4B.

In the configuration illustrated in Fig. 4B, the luer connection 56 does not extend externally or away from the end wall 25. The end wall 25 is formed substantially continuously and/or as one piece with the sidewall 22 of the container 20. The luer connection 56 or appropriate connection can be formed to be substantially flush with the end wall 25 as well. As illustrated in Fig. 4B, the stand 28, as a separate and/or additional member, need not be included to support and/or direct forces during centrifugation onto the container 20.

An internal support member or portion 173 can extend from an internal surface 25a of the end wall 25 to engage and/or support at least a portion of the valve assembly 120. For example, the support portion 173 can engage the first valve body 124 or the second valve body 144. The support portion 173 can assist in supporting and holding the valve assembly 120 during use of the device 10, including during centrifugation of the container and/or the whole material. It is understood, therefore, that in various embodiments other container engagement portions, such as the flange 126, can be eliminated from the valve assembly 120. Also, a cap 175 can be used to cap the luer connection 56, if selected. It is understood, however, that the cap 175 is not necessary and that the luer connection 56 can be maintained through the end wall 25 without the cap 175.

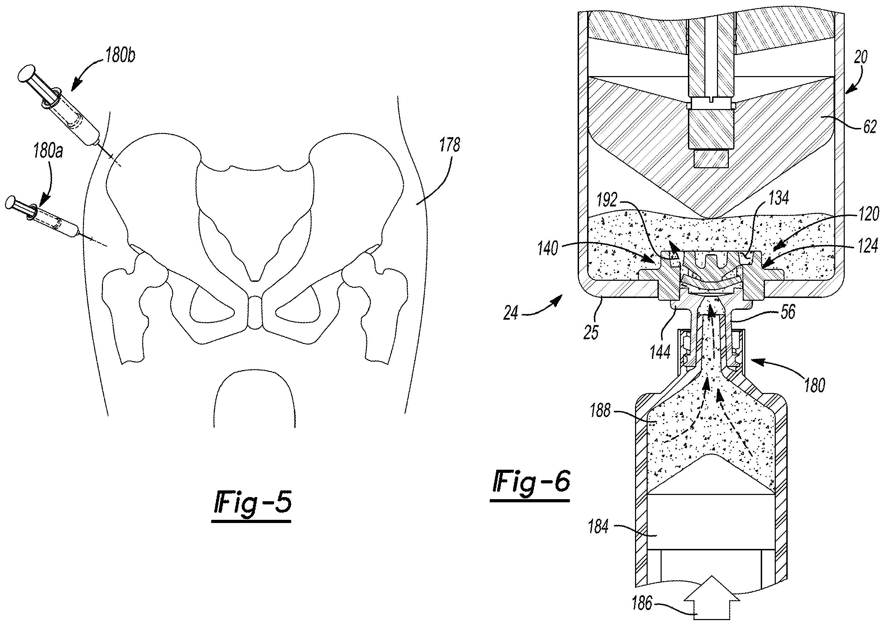

According to the various embodiments, the separation assembly that includes the container 20 can be used to separate a material, such as a biological material collected from a patient 178. Figs. 5-8 illustrate, and are discussed herein, an exemplary method of a use of the container 20. The biological material 188 can include whole blood, bone marrow, adipose tissue, stem cells or multi-potent cells, or other appropriate materials. It is understood that separated materials may also be previously pooled materials. The separation assembly of the container 20 can be used to separate any whole material that can be fractionated, such as with gravity fractionation. Non-biological materials include silts, (e.g. from effluent sources), compound suspensions of industrial waste, etc.

Nevertheless, as illustrated in Figs. 5-8, the separation container can be used to separate a whole material positioned in a container 20 that is withdrawn from the patient 178. A first syringe 180 can be used to withdraw the whole material or whole materials from the patient 178. For example, whole blood, bone marrow aspirate, or a combination of the two can be withdrawn from the patient 178. The single first syringe 180 can be used to withdraw all of the whole materials from the patient 178 or multiple first syringes 180 can be used to withdraw the whole material and all of the whole materials can be positioned within the container 20 sequentially, such that all of the materials are within the container 20 at the same time. For example, whole blood can be withdrawn with the first syringe 180. Also, bone marrow can be aspirated with the same or different first syringe 180a for filling into the container 20.

With reference to Fig. 6, the syringe 180 can interconnect with the luer 56 of the container 20. A plunger 184 of the syringe 180 can be moved generally in the direction of arrow 186 to force the whole material 188 through the valve assembly 120 into the container 20 generally in the direction of arrow 192. When the whole material 188 is being moved in the direction of arrow 192, the valve member 140 can flex away from the valve seat or edge 142 of the second valve body 144 to allow the whole material 188 to pass through the passage 134 of the valve assembly 120. This can allow the container 20 to fill between the first buoy member 62 and the first end wall 25. The buoy 60 can move or be pushed by the whole material 188 as the whole material 188 is moved into the container 20. It is understood, however, that a volume of the whole material 188 may be greater than that of a volume between the buoy 62 and the first end wall 25 of the container 20, especially when the buoy 60 contacts the stop legs 96a-c. Accordingly, a certain volume of material 188 can be forced to pass the buoy member 62, according to various embodiments. It can be selected, however, but is not required to form a seal between the first buoy member 62 towards the second end 26 while being filled and the container 20 such that material will not pass the buoy 60 and that the container 20 will only be filled between the first buoy member 62, or the exterior surface thereof, and the first end wall 25.

Once the container 20 is filled with the selected volume of the whole material 188, which can include a combination of material such as whole blood and bone marrow, the stand 28 can be interconnected with the container 20. As discussed above, the stand 28 can be adhered, such as with glue, or have an interference fit with the cap that is positioned over the luer 56. The stand 28 allows the luer 56 to extend out of the first end wall 25 while maintaining a selected external diameter, such as to fit within a bucket of a centrifuge. Centrifuge assemblies can include an appropriate centrifuge assembly, such as model 755VES centrifuge sold by The Drucker Company of Philipsburg, PA. The stand 28, however, is not required as the check valve assembly can be contained within the container 20 and an opening or flush portion can be provided through the first end wall 25. The stand 28 can support the container within the centrifuge, such as a bucket of the centrifuge to transfer forces to the wall 22 of the container 20. Alternatively, or in addition thereto, the stand 28 can be fixed or formed as one member with the container 20 such that the valve assembly is contained within the stand 28 and a flush opening is provided through the bottom wall 156 of the stand. If the stand 28 is formed as one piece with the container 20 the configuration may be similar to that illustrated in Fig. 4B.

A centrifuge, as is generally understood, can spin the container 20 including the whole material to increase gravitational force within the container 20 to allow for gravitational separation of the whole material 188 based upon the specific gravities or densities of the various components of the whole material. In the centrifuge the gravitational forces are generally applied along a longitudinal axis 20a of the container 20 and towards the first end 24 such that the most dense material is urged towards the first end wall 25 and the port 56. The centrifugation, therefore, causes fractionation of the whole material 188 that is positioned within the container 20. Because the container 20 is filled first between the first end wall 25 and the buoy 60, the container 20 can be positioned in the centrifuge such that the bottom or first end wall 25 is positioned furthest away from a center of rotation. It is understood, as discussed and illustrated in Fig. 7, that some whole material 188 can pass the buoy system 60 during filling. Nevertheless, denser material portions will collect or fractionate nearest the first end wall 25 or at the bottom of the container 20 when positioned in a centrifuge device such that the first end wall is positioned away from the center of rotation of the centrifuge device.

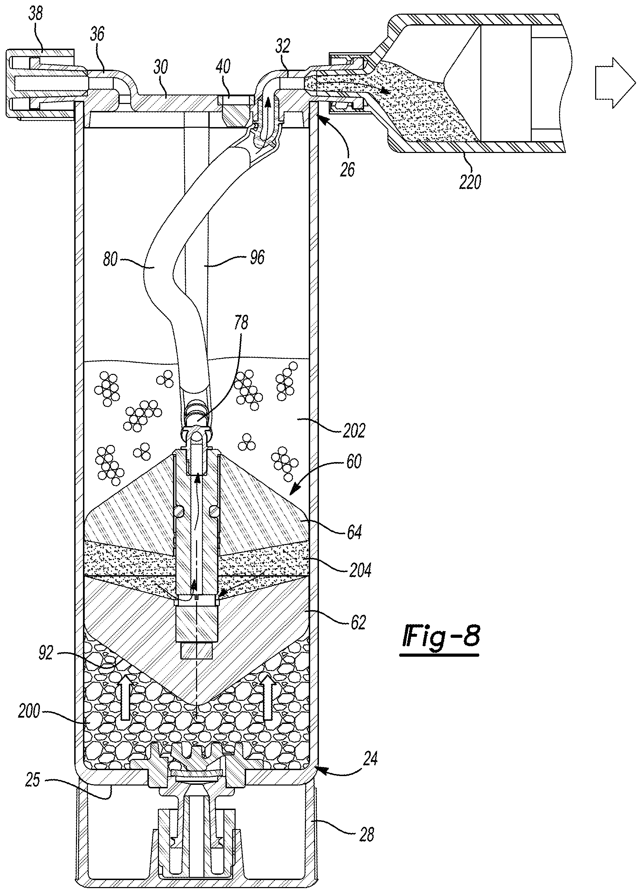

As exemplarily illustrated in Fig. 8, the fractionated whole material, such as whole blood, bone marrow aspirate, or a combination thereof, can include a first fraction 200 of material generally with a density greater than 1.06 g/cc, such as red blood cells, bone spicules, or blood and marrow clots nearest the first end wall 25. The lightest fraction or second fraction 202 can include plasma and other like components of whole blood, bone marrow aspirate, or a combination thereof. A middle or third fraction 204 can include buffy coat including platelets, white blood cells, stromal cells, and other components of whole blood, bone marrow aspirate, or a combination thereof. Since the red blood cells and other dense components are maintained between the buoy 60 and the first end wall 25 any globules of coagulated materials will not interfere with the specific gravity or density of the buoy system 60. For example, any clot that may form in the whole blood, bone marrow aspirate, or a combination thereof, will not interfere with movement of the buoy 60 within the container 20. If a clot contacts the buoy 60 on or around a second buoy component 64 as the buoy 60 attempts to move through the container 20, due to the specific gravity of the buoy 60 relative to the fractioned materials, the clot may alter the effective gravity of the buoy 60 by contacting and sticking to at least the second buoy component 64 and increasing its respective density. The clot, however, in the separation container 20, will remain between the first end wall 25 and the buoy 60 and therefore substantially not interact with the buoy 60 or alter the effective density of the buoy 60. Accordingly, the separation can fractionate the whole material and not be affected by components of the whole material that may alter the density of the buoy 60. Thus, the buoy 60 maintains or substantially maintains its original and intended density throughout the separation. Further, the buoy 60 need not act as an agitator to achieve the selected separation of the whole material based on the gravity separation. After separation, as illustrated in Fig. 8, a second syringe 220 can interconnect with the withdrawal or removal port 32 to withdraw material through the withdrawal tube 80 that is interconnected with the buoy 60 via the port 78 thus the third fraction, such as the buffy coat 204 that is positioned within the separation volume of the buoy 60 can be withdrawn from the second end 26 of the container 20 and the cap 30 that is opposite the first end 24. Thus, a material will be withdrawn from the container 20 at an end that is opposite the first end 24. Therefore, as illustrated in Fig. 6, the container 20 can be filled through the first end 24, such as via the valve assembly 120, and withdrawn from the second end 26 that is opposite the first end 24 and also on an opposite side of the buoy 60, as illustrated in Fig. 8.

Also, materials can be withdrawn through the cap 30 using either of the ports 32 or 36. For example, the port 32 can be used to draw material 204 from between the two members of the buoy 60. Also, the second port 36 can be used to withdraw material 202 from between the buoy 60 and the cap 30.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A system to separate a whole material including multiple components, comprising: a container (20) having an external wall (22) defining a container volume within the external wall and extending along a long axis (20a) between a first end (24) and a second end (26); a separation buoy (60) positioned within the container volume between the first end and the second end; a first fill port (56) near the first end of the container; a valve assembly (120) formed within the first fill port; and a second withdrawal port (32) near the second end of the container; wherein the whole material is operable to enter the container through the first fill port and at least a selected component of the whole material is operable to be withdrawn from the container through the second withdrawal port, and wherein the whole material is whole blood, bone marrow aspirate, or a combination thereof.

2. The system of Claim 1, wherein the valve assembly includes a check valve assembly formed within the first fill port having a flexible valve member and a valve seat, wherein the flexible valve member is moveable from a closed position when the flexible valve member sealingly engages the valve seat to an open position when the flexible valve member at least partially disengages the valve seat.

3. The system of any of Claims 1 or 2, wherein the first end includes a first end wall;
wherein the first fill port includes an extension portion that extends beyond the first end wall.

4. The system of Claim 3, further comprising:
a stand operably engaging the container to support the container on a surface and at least partially enclose the extension portion of the first fill port.

5. The system of Claim 4, wherein the stand includes:
a stand end wall;
an outer stand wall extending from the stand end wall to engage at least one of the first end or the external wall of the container;
an inner stand wall extending from the stand end wall to engage the extension portion of the first fill port.

6. The system of Claim 1, wherein the separation buoy substantially physically separates the first end of the container from the second end of the container;
wherein the first fill port near the first end of the container is positioned in the container relative to the separation buoy to minimize interference of a coagulated material during an operation of the separation buoy.

7. The system of Claim 6, wherein the separation buoy includes at least a first buoy member and a second buoy member configured to define a separation volume between the first buoy member and the second buoy member;
wherein the second withdrawal port is in fluid communication with the separation volume to remove a selected fraction from the container through the second withdrawal port, where the second withdrawal port is separated from the first fill port by at least one of the first buoy member or the second buoy member.

8. The system of Claim 1, further comprising:
a stand operably engaging the container to support the container in a centrifuge device during centrifugation of the container.

9. The system of Claim 1, wherein the first end includes a first end wall;
wherein the first fill port is substantially flush with the first end wall.

10. The system of Claim 1, wherein the separation buoy includes at least a first buoy member and a second buoy member;
wherein the first buoy member includes a first wall having a steep angle to ease passage of at least a portion of the whole material past the separation buoy within the container.

11. The system of Claim 1, wherein the separation buoy includes a buoy density of about 1.01 grams per cubic centimeter to about 1.10 grams per cubic centimeter.

12. A method of separating a whole material, comprising: providing a container (20) having a sidewall (22) extending from a first end (24) to a second end (26); providing a buoy (60) within the container between the first end and the second end; providing a first fill port (56) near the first end of the container; providing a second withdrawal port (32) near the second end of the container; and providing a valve (120) in the first fill port to limit flow of the whole material out of the container; wherein the first fill port is operable to allow filling of the container on a first side of the buoy and the second withdrawal port is operable to allow emptying of the container on a second side of the buoy and wherein the whole material is whole blood, bone marrow aspirate, or a combination thereof.

13. The method of Claim 12, further comprising:
filling the container with the whole material through the first fill port by causing the valve to open;
applying a force to the container and the whole material within the container to fractionate the whole material into at least a first fraction and a second fraction; and withdrawing at least the first fraction from the second withdrawal port.

14. The method of Claim 13, wherein providing a buoy further includes providing a first buoy member and providing a second buoy member;
wherein withdrawing at least the first fraction from the second withdrawal port includes withdrawing at least the first fraction from between the first buoy member and the second buoy member, and
wherein providing a buoy further includes connecting the provided first buoy member and provided second buoy member with a third buoy member, wherein the third buoy member includes a passage through at least a portion of the third buoy member to allow fluid access to a volume between the first buoy member and the second buoy member.

15. The method of Claim 12, further comprising:
filling the container with the whole material through the first fill port at least initially between the first fill port and the buoy;
applying a force to the container along a longitudinal axis of the container such that the first fill port is positioned to allow the whole material within the container to fractionate into at least a first fraction and a second fraction, wherein a coagulated portion remains substantially between the fill port and the buoy while applying the force.

## Patentansprüche

1. System zum Trennen eines Vollmaterials, das mehrere Komponenten beinhaltet, umfassend: einen Behälter (20) mit einer externen Wand (22), die ein Behältervolumen innerhalb der externen Wand definiert und sich entlang einer Längsachse (20a) zwischen einem ersten Ende (24) und einem zweiten Ende (26) erstreckt; einem Trennschwimmer (60), der innerhalb des Behältervolumens zwischen dem ersten Ende und dem zweiten Ende positioniert ist; einer ersten Füllöffnung (56) nahe des ersten Endes des Behälters; einer Ventilgruppe (120), die innerhalb der ersten Füllöffnung geformt ist; und einer zweiten Ablassöffnung (32) nahe des zweiten Endes des Behälters; wobei das Vollmaterial geeignet ist, um durch die erste Füllöffnung in den Behälter einzutreten, und mindestens eine ausgewählte Komponente des Vollmaterials geeignet, um durch die zweite Ablassöffnung aus dem Behälter abgelassen zu werden, und wobei das Vollmaterial Vollblut, Knochenmarksaspirat oder eine Kombination daraus ist.

2. System nach Anspruch 1, wobei die Ventilgruppe eine innerhalb der ersten Füllöffnung geformte Rückschlagventilgruppe mit einem flexiblem Ventilelement und einem Ventilsitz beinhaltet, wobei das flexible Ventilelement aus einer geschlossenen Position, wenn das flexible Ventilelement den Ventilelement dichtend in Eingriff nimmt, in eine offene Position, wenn das flexible Ventilelement den Ventilsitz mindestens teilweise außer Eingriff lässt, bewegt werden kann.

3. System nach einem der Ansprüche 1 oder 2, wobei das erste Ende eine erste Endwand beinhaltet;
wobei die erste Füllöffnung einen Erweiterungsteil beinhaltet, der sich über die erste Endwand hinaus erstreckt.

4. System nach Anspruch 3, ferner umfassend:
einen Ständer, der den Behälter operativ in Eingriff nimmt, um den Behälter auf einer Oberfläche zu stützen und den Erweiterungsteil der ersten Füllöffnung mindestens teilweise zu umschließen.

5. System nach Anspruch 4, wobei der Ständer beinhaltet: eine Ständerendwand;
eine Ständeraußenwand, die sich von der Ständerendwand erstreckt, um mindestens eines des ersten Endes oder der externen Wand des Behälters in Eingriff zu nehmen;
eine Ständerinnenwand, die sich von der Ständerendwand erstreckt, um den Erweiterungsteil der ersten Füllöffnung in Eingriff zu nehmen.

6. System nach Anspruch 1, wobei der Trennschwimmer im Wesentlichen das erste Ende des Behälters vom zweiten Ende des Behälters physisch trennt;
wobei die erste Füllöffnung nahe des ersten Endes des Behälters im Behälter relativ zum Trennschwimmer positioniert ist, um eine Interferenz eines koagulierten Materials während eines Betriebs des Trennschwimmers zu minimieren.

7. System nach Anspruch 6, wobei der Trennschwimmer mindestens ein erstes Schwimmerelement und ein zweites Schwimmerelement beinhaltet, die konfiguriert sind, um ein Trennvolumen zwischen dem ersten Schwimmerelement und dem zweiten Schwimmerelement zu definieren;
wobei sich die zweite Ablassöffnung in Fluidverbindung zum Trennvolumen befindet, um eine ausgewählte Fraktion aus dem Behälter durch die zweite Ablassöffnung zu entfernen, wobei die zweite Ablassöffnung durch mindestens eines des ersten Schwimmerelements oder des zweiten Schwimmerelements von der ersten Füllöffnung getrennt ist.

8. System nach Anspruch 1, ferner umfassend:
einen Ständer, der den Behälter operativ in Eingriff nimmt, um den Behälter während des Zentrifugierens des Behälters in einer Zentrifugiervorrichtung zu stützen.

9. System nach Anspruch 1, wobei das erste Ende eine erste Endwand beinhaltet, wobei die erste Füllöffnung im Wesentlichen mit der ersten Endwand bündig ist.

10. System nach Anspruch 1, wobei der Trennschwimmer mindestens ein erstes Schwimmerelement und ein zweites Schwimmerelement beinhaltet;
wobei das erste Schwimmerelement eine erste Wand mit einem Schwenkwinkel beinhaltet, um das Passieren mindestens eines Teils des Vollmaterials innerhalb des Behälters am Trennschwimmer vorbei zu erleichtern.

11. System nach Anspruch 1, wobei der Trennschwimmer eine Schwimmerdichte von etwa 1,01 Gramm je Kubikzentirmeter bis etwa 1,10 Gramm je Kubikzentimeter beinhaltet.

12. Verfahren zum Trennen eines Vollmaterials, umfassend: ein Bereitstellen eines Behälters (20) mit einer Seitenwand (22), die sich von einem ersten Ende (24) zu einem zweiten Ende (26) erstreckt; ein Bereitstellen eines Schwimmers (60) innerhalb des Behälters zwischen dem ersten Ende und dem zweiten Ende; ein Bereitstellen einer ersten Füllöffnung (56) nahe des ersten Endes des Behälters; ein Bereitstellen einer zweiten Ablassöffnung (32) nahe des zweiten Endes des Behälters; und ein Bereitstellen eines Ventils (120) in der ersten Füllöffnung, um den Strom des Vollmaterials aus dem Behälter zu begrenzen; wobei die erste Füllöffnung geeignet ist, um ein Füllen des Behälters auf einer ersten Seite des Schwimmers zuzulassen, und die zweite Ablassöffnung geeignet ist, um ein Entleeren des Behälters auf einer zweiten Seite des Schwimmers zuzulassen, und wobei das Vollmaterial Vollblut, Knochenmarksaspirat oder eine Kombination daraus ist.

13. Verfahren nach Anspruch 12, ferner umfassend:
ein Füllen des Behälters mit dem Vollmaterial durch die erste Füllöffnung, indem bewirkt wird, dass sich das Ventil öffnet;
ein Anwenden einer Kraft auf den Behälter und das Vollmaterial im Behälter, um das Vollmaterial in mindestens eine erste Fraktion und eine zweite Fraktion zu fraktionieren; und ein Ablassen mindestens der ersten Fraktion aus der zweiten Ablassöffnung.

14. Verfahren nach Anspruch 13, wobei das Bereitstellen eines Schwimmers ferner ein Bereitstellen eines ersten Schwimmerelements und ein Bereitstellen eines zweiten Schwimmerelements beinhaltet;
wobei das Ablassen mindestens der ersten Fraktion aus der zweiten Ablassöffnung ein Ablassen mindestens der ersten Fraktion aus zwischen dem ersten Schwimmerelement und dem zweiten Schwimmerelement beinhaltet, und
wobei das Bereitstellen eines Schwimmers ferner ein Verbinden des bereitgestellten ersten Schwimmerelements und des bereitgestellten zweiten Schwimmerelements mit einem dritten Schwimmerelement beinhaltet, wobei das dritte Schwimmerelement einen Durchgang durch mindestens einen Teil des dritten Schwimmerelements beinhaltet, um zuzulassen, dass Fluid in ein Volumen zwischen dem ersten Schwimmerelement und dem zweiten Schwimmerelement dringt.

15. Verfahren nach Anspruch 12, ferner umfassend:
ein Füllen des Behälters mit dem Vollmaterial durch die erste Füllöffnung mindestens initial zwischen der ersten Füllöffnung und dem Schwimmer;
ein Anwenden einer Kraft auf den Behälter entlang einer Längsachse des Behälters, so dass die erste Füllöffnung positioniert ist, um zuzulassen, dass das Vollmaterial in mindestens eine erste Fraktion und eine zweite Fraktion fraktioniert wird, wobei ein koagulierter Teil im Wesentlichen zwischen der Füllöffnung und dem Schwimmer bleibt, während die Kraft angewandt wird.

## Revendications

1. Système pour séparer une substance formant un tout comprenant de multiples composants, comprenant : un récipient (20) possédant une paroi externe (22) définissant un volume de récipient à l'intérieur de la paroi externe et s'étendant le long d'un axe long (20a) entre une première extrémité (24) et une seconde extrémité (26) ; une bouée de séparation (60) positionnée dans le volume du récipient entre la première extrémité et la seconde extrémité ; un premier orifice de remplissage (56) près de la première extrémité du récipient ; un ensemble de soupape (120) formé à l'intérieur du premier orifice de remplissage ; et un second orifice de retrait (32) près de la seconde extrémité du récipient ; ladite substance formant un tout pouvant entrer dans le récipient par le premier orifice de remplissage et au moins un composant sélectionné de la substance formant un tout pouvant être retiré du récipient par le second orifice de retrait, et ladite substance formant un tout étant du sang total, de l'aspirat de moelle osseuse, ou une combinaison de ceux-ci.

2. Système selon la revendication 1, ledit ensemble de soupape comprenant un ensemble de clapet anti-retour formé à l'intérieur du premier orifice de remplissage possédant un élément de soupape souple et un siège de soupape, ledit élément de soupape souple étant mobile à partir d'une position fermée lorsque l'élément de soupape souple se met en prise de manière étanche avec le siège de soupape jusque dans une position ouverte lorsque l'élément de soupape souple se sépare au moins partiellement du siège de soupape.

3. Système selon l'une quelconque des revendications 1 ou 2, ladite première extrémité comprenant une première paroi d'extrémité ;
ledit premier orifice de remplissage comprenant une partie d'extension qui s'étend au-delà de la première paroi d'extrémité.

4. Système selon la revendication 3, comprenant en outre :
un support se mettant en prise fonctionnellement avec le récipient pour supporter le récipient sur une surface et enfermer au moins partiellement la partie d'extension du premier orifice de remplissage.

5. Système selon la revendication 4, ledit support comprenant : une paroi d'extrémité de support ;
une paroi de support externe s'étendant à partir de la paroi d'extrémité de support pour se mettre en prise avec au moins l'une de la première extrémité ou de la paroi externe du récipient ;
une paroi de support interne s'étendant à partir de la paroi d'extrémité de support pour se mettre en prise avec la partie d'extension du premier orifice de remplissage.

6. Système selon la revendication 1, ladite bouée de séparation séparant sensiblement de manière physique la première extrémité du récipient de la seconde extrémité du récipient ;
ledit premier orifice de remplissage près de la première extrémité du récipient étant positionné dans le récipient par rapport à la bouée de séparation pour minimiser l'interférence d'une substance coagulée durant le fonctionnement de la bouée de séparation.

7. Système selon la revendication 6, ladite bouée de séparation comprenant au moins un premier élément de bouée et un deuxième élément de bouée configuré pour définir un volume de séparation entre le premier élément de bouée et le deuxième élément de bouée ;
ledit second orifice de retrait étant en communication fluidique avec le volume de séparation pour retirer une fraction sélectionnée du récipient par le second orifice de retrait, où le second orifice de retrait est séparé du premier orifice de remplissage par au moins l'un du premier élément de bouée ou du deuxième élément de bouée.

8. Système selon la revendication 1, comprenant en outre :
un support se mettant en prise fonctionnellement avec le récipient pour supporter le récipient dans un dispositif de centrifugation pendant la centrifugation du récipient.

9. Système selon la revendication 1, ladite première extrémité comprenant une première paroi d'extrémité ; ledit premier orifice de remplissage affleurant sensiblement la première paroi d'extrémité.

10. Système selon la revendication 1, ladite bouée de séparation comprenant au moins un premier élément de bouée et un deuxième élément de bouée ;
ledit premier élément de bouée comprenant une première paroi possédant un angle aigu pour faciliter le passage d'au moins une partie de la substance formant un tout au-delà de la bouée de séparation à l'intérieur du récipient.

11. Système selon la revendication 1, ladite bouée de séparation comprenant une densité de bouée allant d'environ 1,01 gramme par centimètre cube à environ 1,10 gramme par centimètre cube.

12. Procédé de séparation d'une substance formant un tout, comprenant : la fourniture d'un récipient (20) possédant une paroi latérale (22) s'étendant à partir d'une première extrémité (24) jusqu'à une seconde extrémité (26) ; la mise en place d'une bouée (60) à l'intérieur du récipient entre la première extrémité et la seconde extrémité ; l'aménagement d'un premier orifice de remplissage (56) près de la première extrémité du récipient ; l'aménagement d'un second orifice de retrait (32) près de la seconde extrémité du récipient ; et la fourniture d'une soupape (120) dans le premier orifice de remplissage pour limiter l'écoulement de la substance formant un tout hors du récipient ; ledit premier orifice de remplissage étant utilisable pour permettre le remplissage du récipient sur un premier côté de la bouée et ledit second orifice de retrait étant utilisable pour permettre la vidange du récipient sur un second côté de la bouée et ladite substance formant un tout étant du sang total, de l'aspirat de moëlle osseuse, ou une combinaison de ceux-ci.

13. Procédé selon la revendication 12, comprenant en outre :
le remplissage du récipient avec la substance formant un tout par le premier orifice de remplissage en amenant la soupape à s'ouvrir ;
l'application d'une force au récipient et à la substance formant un tout à l'intérieur du récipient pour fractionner la substance formant un tout en au moins une première fraction et une seconde fraction ; et le retrait d'au moins la première fraction par le second port de retrait.

14. Procédé selon la revendication 13, ladite mise en place d'une bouée comprenant en outre la mise en place d'un premier élément de bouée et la mise en place d'un deuxième élément de bouée ;
ledit retrait d'au moins la première fraction par le second orifice de retrait comprenant le retrait d'au moins la première fraction de l'espace entre le premier élément de bouée et le deuxième élément de bouée, et
ladite mise en place d'une bouée comprenant en outre le raccord du premier élément de bouée mis en place et du deuxième élément de bouée mise en place avec un troisième élément de bouée, ledit troisième élément de bouée comprenant un passage à travers au moins une partie du troisième élément de bouée pour permettre au fluide d'accéder à un volume entre le premier élément de bouée et le deuxième élément de bouée.

15. Procédé selon la revendication 12, comprenant en outre :
le remplissage du récipient avec la substance formant un tout par le premier orifice de remplissage au moins initialement entre le premier orifice de remplissage et la bouée ;
l'application d'une force au récipient le long d'un axe longitudinal du récipient de sorte que le premier orifice de remplissage soit positionné pour permettre à la substance formant un tout à l'intérieur du récipient de se fractionner en au moins une première fraction et une seconde fraction, une partie coagulée restant sensiblement entre l'orifice de remplissage et la bouée pendant l'application de la force.
